(19) Europäisches Patentamt
European Patent Office
Office européen des brevets

(11) Veröffentlichungsnummer: **0 156 197**
**B1**

(12) # EUROPÄISCHE PATENTSCHRIFT

(45) Veröffentlichungstag der Patentschrift: **07.06.89**

(51) Int. Cl.$^4$: **C 07 D231/06**, D 06 L 3/12

(21) Anmeldenummer: **85102443.0**

(22) Anmeldetag: **05.03.85**

(54) **Pyrazolinverbindungen.**

(30) Priorität: **15.03.84 DE 3409429**

(43) Veröffentlichungstag der Anmeldung: **02.10.85 Patentblatt 85/40**

(45) Bekanntmachung des Hinweises auf die Patenterteilung: **07.06.89 Patentblatt 89/23**

(84) Benannte Vertragsstaaten: **CH DE FR GB IT LI**

(56) Entgegenhaltungen:
**DE-A- 2 011 552**
**DE-A- 2 700 996**
**DE-A- 3 134 942**
**GB-A- 1 013 454**
**GB-A- 1 204 953**
**Die Akte enthält technische Angaben, die nach dem Eingang der Anmeldung eingereicht wurden und die nicht in dieser Patentschrift enthalten sind.**

(73) Patentinhaber: **BAYER AG**
**D-5090 Leverkusen 1 Bayerwerk (DE)**

(72) Erfinder: **Schellhammer, Carl-Wolfgang, Dr.**
**Katharinental 26**
**D-5060 Bergisch-Gladbach 2 (DE)**
Erfinder: **Seng, Florin, Dr.**
**Am Katterbach 46**
**D-5060 Bergisch-Gladbach 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Als optische Aufheller vervendbare 1-(Dialkylaminoalkylsulfonylphenyl)-3-phenyl-2-pyrazoline sind unter anderem aus US-A-3 131 079, GB-A-1 013 454, DE-A-2 700 996 und DE-A-3 134 942 bekannt.

Gegenstand der Erfindung sind Verbindungen der Formel

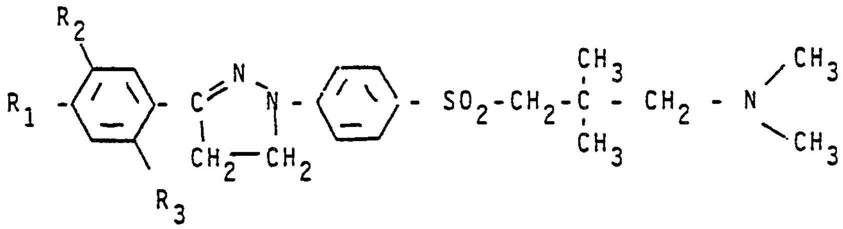

(I)

worin

$R_1$ und $R_2$ H oder Cl und

$R_3$ H oder $CH_3$ bedeuten,

deren Hydrochloride sowie deren Quaternierungsprodukte der Formel

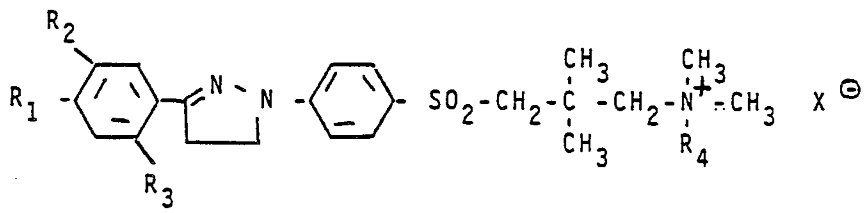

(Ia)

worin

$R_1$-$R_3$ die obengenannte Bedeutung haben,

$R_4$ für $CH_3$ oder $C_2H_5$ und

X für $CH_3OSO_3^-$, $C_2H_5OSO_3^-$ oder

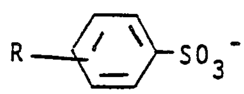

(R = H, Cl oder $CH_3$)

stehen.

Die neuen Pyrazolinverbindungen können beispielsweise dadurch hergestellt werden, daß man in an sich bekannter Weise Ketonverbindungen der Formel

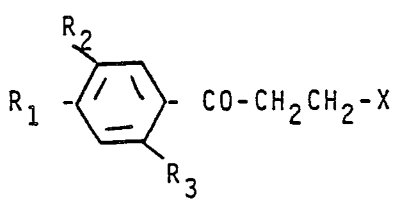

(II)

worin X eine Abgangsgruppe ist, mit Hydrazinverbindungen der Formel

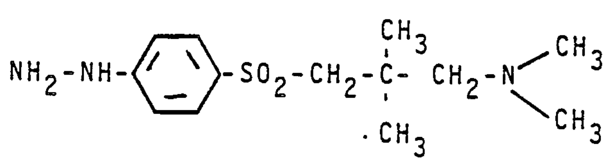

(III)

umsetzt und das Reaktionsprodukt gewünschtenfalls quaterniert.

Geeignete Abgangsgruppen X sind Halogenatome und Dialkylaminogruppen.

Die neuen Pyrazolinverbindungen zeigen in gelöstem oder feinverteiltem Zustand eine starke Fluoreszenz und eignen sich hervorragend als optische Aufheller. Während die « neutralen » Verbindungen insbesondere zum Weißtönen von Textilmaterialien aus hydrophoben Fasern, insbesondere Polyamidfasern eingesetzt werden, dienen die bevorzugten quaternierten Produkte zum Aufhellen von sauermodifizierten Fasermaterialien, wie z. B. Acrylfasern, nach üblichen Färbemethoden und zum Aufhellen von Spinnmassen.

Die quaternierten Verbindungen zeichnen sich gegenüber konstitutionell nächstvergleichbaren bekannten Verbindungen, wie sie z. B. in DE-A-2 700 996 und US-A-3 131 079 beschrieben sind, durch eine verbesserte Lagerungsbeständigkeit ihrer wäßrigen Lösungen aus.

## Beispiel 1

(a) 4-(3-Dimethylamino-2,2-dimethyl-propylsulfonyl)-anilin

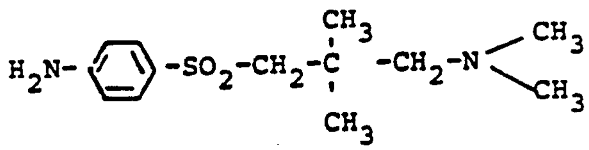

110 g 4-Acetylaminobenzolsulfinsäure werden in 375 ml Methylglykol zusammen mit 46 g Natriumhydrogencarbonat, 25 ml Wasser und 82,5 g 3-Dimethylamino-2,2-dimethylpropylchlorid 2,5 Stunden bei 115 °C gerührt. Anschließend gießt man auf 200 ml Wasser, versetzt mit 200 ml 37 %iger Salzsäure und rührt 2 Stunden bei 100 °C. Nach dem Abkühlen wird mit Natronlauge alkalisch (pH 9-10) gestellt. Dabei scheidet sich das 4-(3-Dimethylamino-2,2-dimethyl-propylsulfonyl)-anilin kristallin ab. Ausbeute 124 g (82 %) Fp : 132 °C

(b) 4-(3-Dimethylamino-2,2-dimethyl-propylsulfonyl)-phenyl-hydrazin

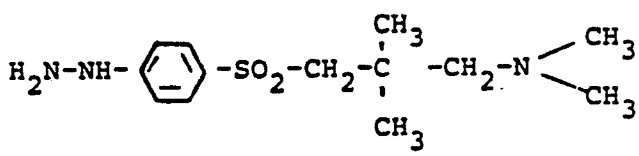

88 g 4-(3-Dimethylamino-2,2-dimethyl-propylsulfonyl)-anilin werden in einer Mischung aus 488 ml Wasser und 98 ml 37 %iger Salzsäure gelöst und bei 0 °C bis 5 °C mit 22,7 g Natriumnitrit, gelöst in 60 ml Wasser, diazotiert. Die erhaltene Lösung des Diazoniumsalzes läßt man innerhalb einer Stunde bei 0 bis 8 °C in eine Mischung aus 245 ml Wasser und 245 ml 40 %iger Natriumbisulfitlösung fließen, wobei man durch Zugabe von insgesamt 51 g 45 %iger Natronlauge den pH-Wert bei 7 hält. Anschließend rührt man 1 Stunde bei Raumtemperatur, gibt dann 115 g 37 %ige Salzsäure zu und rührt eine weitere Stunde bei 100 °C. Zur Entfernung des Schwefeldioxides leitet man während des Abkühlens 4 Stunden lang gasförmigen Stickstoff durch den Ansatz. Danach wird mit 200 g 45 %iger Natronlauge alkalisch gestellt und das dabei abgeschiedene 4-(3-Dimethylamino-2,2-dimethylpropylsulfonyl)-phenylhydrazin abgesaugt. Man erhält 83 g (91 %) Hydrazin, das nach dem Umlösen aus Toluol/Tonsil bei 120-21 °C schmilzt.

## Beispiel 2

1-(3-Dimethylamino-2,2-dimethyl)-propylsulfonyl-phenylhydrazin werden zusammen mit 20,3 g 3-chlor-1-(4-chlorphenyl)-propanon (1) in 150 ml Glykolmonomethylether und 12,5 ml Essigsäure 15 Stunden bei 115 °C gerührt. Anschließend wird das Lösungsmittel bei einer Badtemperatur von 50 °C im Wasserstrahlvakuum abdestilliert und der Rückstand in 100 ml Wasser aufgenommen. Die klare Lösung neutralisiert man mit Natriumbicarbonat. Dabei scheiden sich 29 g (67 %) 1-[4-(3-Dimethylamino-2,2-dimethyl-propyl-sulfonyl-phenyl)]-3-(4-chlorphenyl)-pyrazolin (2) als gelbe Kristalle ab, die nach dem Umlösen aus Ethanol/A-Kohle bei 132 °C schmelzen. $\lambda_{max}$ = 370 nm.

Analog wurden folgende Pyrazoline hergestellt

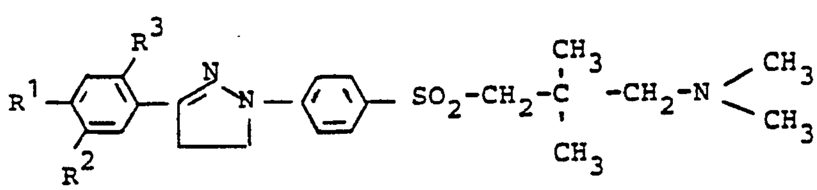

| | $R^1$ | $R^2$ | $R^3$ | Fp | $\lambda_{max}$ (in DMF) |
|---|---|---|---|---|---|
| 3) | H | H | H | 112°C | 364 nm |
| 4) | Cl | Cl | H | 148°C | 374 nm |
| 5) | Cl | Cl | $CH_3$ | 152°C | 370 nm |

Beispiel 6

1-[4-(3-Dimethylamino-2,2-dimethyl-propyl-sulfonyl-phenyl)]-3-(4-chlorphenyl)-pyrazolin(2)-methosulfat.

4,4 g 1-[4-(3-Dimethylamino-2,2-dimethyl-propyl-sulfonyl-phenyl)]-3-(4-chlorphenyl)-pyrazolin(2) werden in 70 ml Chlorbenzol gelöst, mit 2 g Dimethylsulfat versetzt und bei Raumtemperatur gerührt. Nach einer Stunde beginnt ein Niederschlag auszufallen. Man erwärmt nun auf 50 °C und rührt noch 4 Stunden. Anschließend wird auf Raumtemperatur abgekühlt und abgesaugt. Man erhält 5,6 g (100 %) 1-[4-(3-Trimethylammonium-2,2-dimethyl-propyl-sulfonyl-phenyl)]-3-(4-chlorphenyl)-pyrazolin(2)-methosulfat in Form gelber Kristalle, die bei 158 °C schmelzen $\lambda_{max} = 369$ nm.

Analog wurden folgende Pyrazoline hergestellt

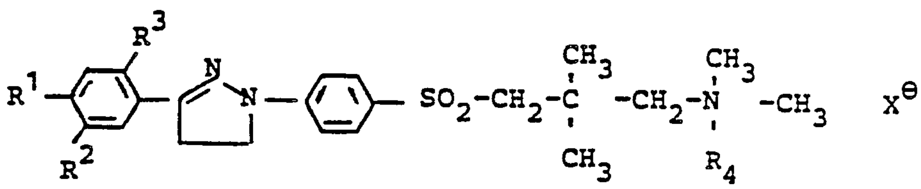

| | $R^1$ | $R^2$ | $R^3$ | $R^4$ | X | $\lambda_{max}$ (in DMF) |
|---|---|---|---|---|---|---|
| 7) | H | H | H | $CH_3$ | $H_3COSO_3^{\ominus}$ | 364 nm |
| 8) | Cl | H | H | $C_2H_5$ | $H_5C_2OSO_3^{\ominus}$ | 370 nm |
| 9) | Cl | H | H | $CH_3$ | 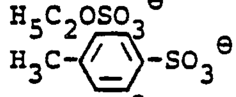 | 370 nm |
| 10) | Cl | Cl | H | $CH_3$ | $H_3COSO_3^{\ominus}$ | 374 nm |
| 11) | Cl | Cl | $CH_3$ | $CH_3$ | $H_3COSO_3^{\ominus}$ | 369,5 nm |

## Patentansprüche

1. Pyrazolinverbindungen der Formel

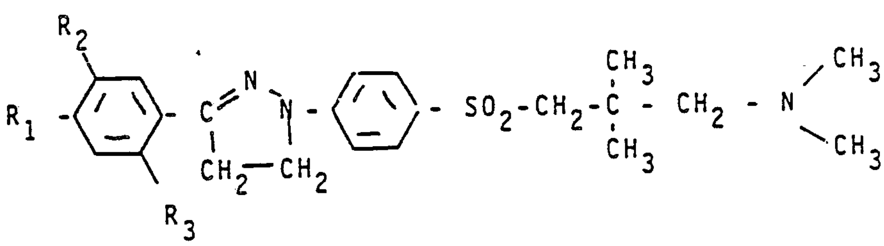

worin

$R_1$ und $R_2$ H oder Cl und

$R_3$ H oder $CH_3$ bedeuten,

deren Hydrochloride sowie deren Quaternierungsprodukte der Formel

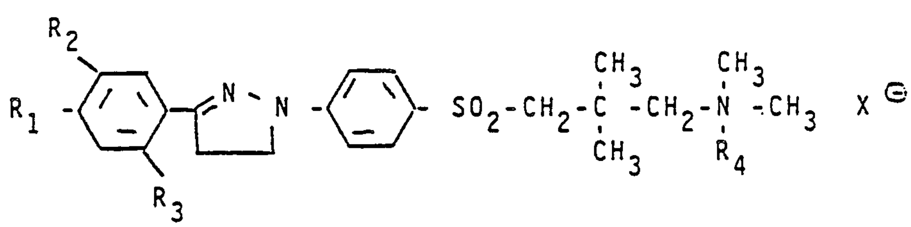

worin

$R_1$-$R_3$ die obengenannte Bedeutung haben,

$R_4$ für $CH_3$ oder $C_2H_5$ und
X für $CH_3OSO_3^-$, $C_2H_5OSO_3^-$ oder

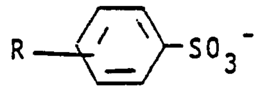 (R = H, Cl oder $CH_3$)

stehen.

2. Pyrazolinverbindung gemäß Anspruch 1 der Formel

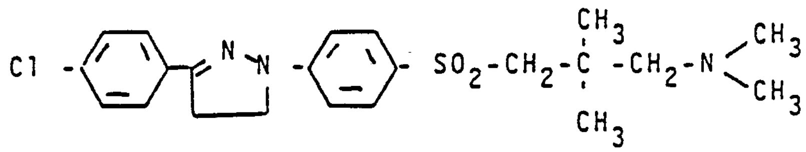

sowie die Quaternierungsprodukte der dort angegebenen Art.

3. Verwendung der Pyrazolinverbindungen gemäß Ansprüchen 1-2 als Weißtöner.

**Claims**

1. Pyrazoline compounds of the formula

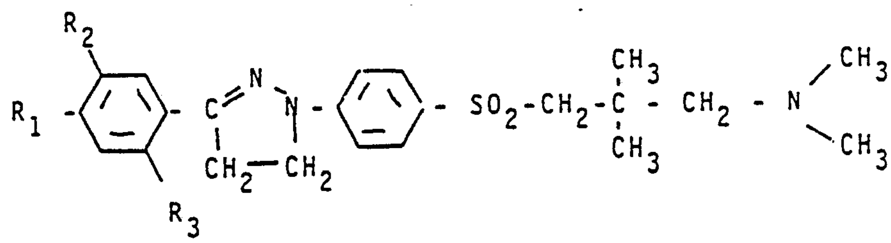

wherein
$R_1$ and $R_2$ denote H or Cl and
$R_3$ denotes H or $CH_3$,
hydrochlorides thereof and quaternization products thereof of the formula

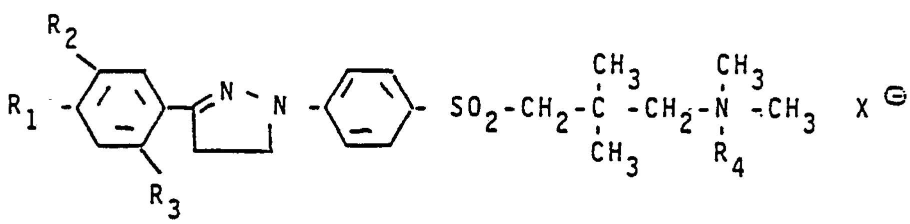

wherein
$R_1$-$R_3$ have the abovementioned meaning,
$R_4$ stands for $CH_3$ or $C_2H_5$ and
X stands for $CH_3OSO_3^-$, $C_2H_5OSO_3^-$ or

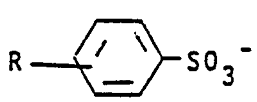 (R = H, Cl or $CH_3$)

2. Pyrazoline compound according to Claim 1, of the formula

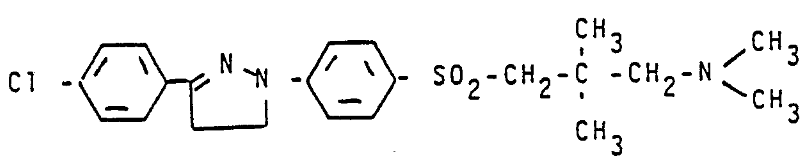

and the quaternization products of the type indicated there.

3. Use of the pyrazoline compounds according to Claims 1-2 as whiteners.

**Revendications**

1. Composés de pyrazoline de formule

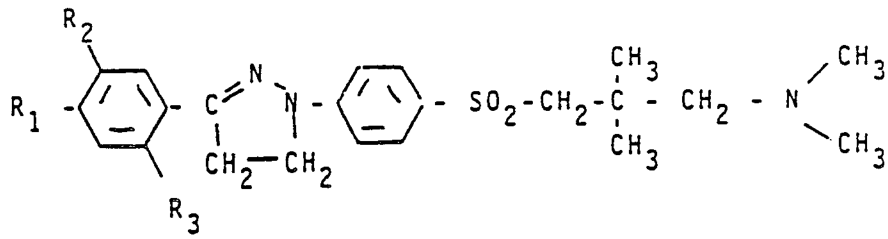

dans laquelle
$R_1$ et $R_2$ représentent H ou Cl et
$R_3$ représente H ou $CH_3$,
leurs chlorhydrates ainsi que leurs produits de quaternisation de formule

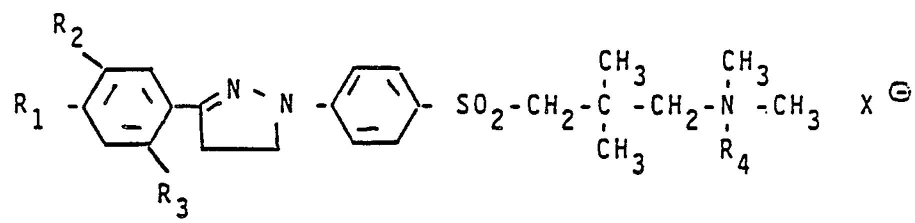

dans laquelle
$R_1$ à $R_3$ ont la définition mentionnée ci-dessus,
$R_4$ représente $CH_3$ ou $C_2H_5$ et
X représente $CH_3OSO_3^-$, $C_2H_5OSO_3^-$ ou

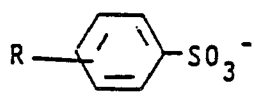 (R = H, Cl ou $CH_3$)

2. Composé de pyrazoline suivant la revendication 1, de formule

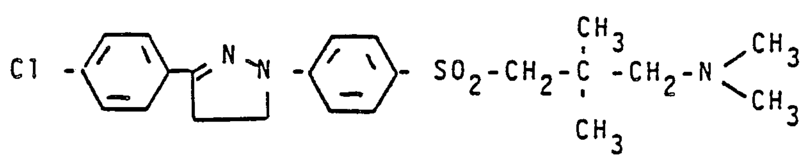

ainsi que les produits de quaternisation du type qui y est indiqué.

3. Utilisation des composés de pyrazoline suivant les revendications 1 et 2 comme azurants optiques.